(19) **Europäisches Patentamt**

**European Patent Office**

**Office européen des brevets**

(11) **EP 0 743 515 B1**

(12) **EUROPÄISCHE PATENTSCHRIFT**

(45) Veröffentlichungstag und Bekanntmachung des Hinweises auf die Patenterteilung:
**02.01.2004 Patentblatt 2004/01**

(51) Int Cl.[7]: **G01N 27/12**, G01N 27/16

(21) Anmeldenummer: **96106973.9**

(22) Anmeldetag: **03.05.1996**

(54) **Verfahren zum Nachweis von Methan in einem Gasgemisch**

Method for detection of methane in a gaseous mixture

Procédé pour la détection de méthane dans un mélange gazeux

(84) Benannte Vertragsstaaten:
**CH DE FR GB LI NL**

(30) Priorität: **19.05.1995 DE 19518547**

(43) Veröffentlichungstag der Anmeldung:
**20.11.1996 Patentblatt 1996/47**

(73) Patentinhaber: **EPCOS AG**
**81669 München (DE)**

(72) Erfinder:
- **Fleischer, Maximilian, Dr.**
  **85635 Höhenkirchen (DE)**
- **Meixner, Hans, Prof.-Dr.**
  **85540 Haar (DE)**

(74) Vertreter: **Epping, Wilhelm, Dipl.-Ing. et al**
**Epping Hermann & Fischer**
**Ridlerstrasse 55**
**80339 München (DE)**

(56) Entgegenhaltungen:
EP-A- 0 603 945  DE-A- 4 310 914
US-A- 4 535 315  US-A- 4 840 913
US-A- 5 374 400

- **PATENT ABSTRACTS OF JAPAN vol. 006, no. 245 (P-159), 3.Dezember 1982 & JP-A-57 141543 (HITACHI SEISAKUSHO KK), 1.September 1982,**
- **PATENT ABSTRACTS OF JAPAN vol. 006, no. 225 (P-154), 10.November 1982 & JP-A-57 127839 (FUIGARO GIKEN KK), 9.August 1982,**
- **PATENT ABSTRACTS OF JAPAN vol. 012, no. 436 (P-787), 17.November 1988 & JP-A-63 165746 (AICHI TOKEI DENKI CO LTD), 9.Juli 1988,**
- **PATENT ABSTRACTS OF JAPAN vol. 007, no. 125 (P-200), 31.Mai 1983 & JP-A-58 042962 (MATSUSHITA DENKO KK), 12.März 1983,**

# Beschreibung

## 1. Einleitung und Stand der Technik

Für die kontinuierliche Überwachung der Konzentration von Methan in Luft werden vorwiegend Gassens oren auf der Basis halbleitender Metalloxide und die als Pellistoren bezeichneten Reaktionswärme- oder Wärmetönungssensoren eingesetzt.

Das am häufigsten als Sensormaterial verwendete Metalloxid ist das bei höheren Temperaturen halbleitende Zinnoxid. Es läßt sich durch eine geeignete Dotierung für bestimmte Gase sensibilisieren, wobei ein aus Pt-dotiertem Zinnoxid bestehendes Sensorelement auf Methan und andere reduzierende Gase anspricht. Die Betriebstemperatur des Sensors beträgt typischerweise etwa T = 350 °C.

Der aus /1/ und /2/ bekannte Methansensor besitzt eine $Ga_2O_3$-Dünnschicht als gasempfindliches Element. Bei Temperaturen T > 700 °C erzeugt der Sensor auch dann noch ein sehr großes Meßsignal, wenn die Methankonzentration weit unterhalb der Explosionsgrenze von 5 % liegt. Selbst wenn die Luft einen hohen Anteil von Wasserdampf enthält, spricht der Sensor nicht an. Auch eine deutlich über dem zulässigen MAK-Wert liegende Konzentration von 1000 ppm Kohlenmonoxid beeinflußt das Meßsignal nur unwesentlich. Wie alle bekannte $SnO_2$-Sensoren und Pellistoren reagiert der $Ga_2O_3$-Sensor äußerst empfindlich auf Alkohole, wobei dieser als Querempfindlichkeit bezeichnete Effekt das durch Methan hervorgerufene Meßsignal unter Umständen völlig überdeckt.

## 2. Ziele und Vorteile der Erfindung

Ziel der Erfindung ist die Schaffung eines Verfahrens, mit dem sich Methan in einem Lösungsmitteldämpfe enthaltenden Gasgemisch auch in geringen Konzentrationen eindeutig nachweisen läßt. Ein Verfahren mit den in Patentanspruch 1 angegebenen Merkmalen besitzt diese Eigenschaft. Die abhängigen Ansprüche betreffen Ausgestaltungen und Weiterbildungen des Verfahrens.

Die Erfindung ermöglicht den Bau von Gaswarngeräten, mit deren Hilfe man die Konzentration von Methan in Luft kontinuierlich überwachen kann. Solche Geräte lassen sich insbesondere in Bereichen einsetzen, wo bekannte Methansensoren aufgrund der in der Luft vorhandenen Alkohol- und/oder Lösungsmitteldämpfe häufig Fehlalarm auslösen.

## 3. Zeichnungen

Die Erfindung wird in folgenden anhand der Zeichnungen erläutert. Hierbei zeigt:

Figur 1 den erfindungsgemäßen Methansensor (ohne Deckschicht) in perspektifischer Darstellung;

Figur 2 die Kammelektroden des Methansensors in Draufsicht;

Figur 3 die Widerstandsheizung des Methansensors in Draufsicht;

Figur 4 einen Schnitt durch den gassensitiven Bereich und die der Aufheizung des Meßgases dienende Deckschicht des Methansensors;

Figur. 5 die Sensitivität einer $Ga_2O_3$-Sensorschicht auf

    a) Alkohol und
    b) Aceton;

Figur 6 die Alkoholempfindlichkeit einer

    a) unbehandelten $Ga_2O_3$-Sensorschicht bzw.
    b) eine mit einer Pt-Dispersion versehenen $Ga_2O_3$-Sensorschicht;

Figur 7 die $CH_4$-Empfindlichkeit der $Ga_2O_3$-Sensorschicht;

Figur 8 die CO-Querempfindlichkeit der $Ga_2O_3$-Sensorschicht.

## 4. Ausführungsbeispiele der Erfindung

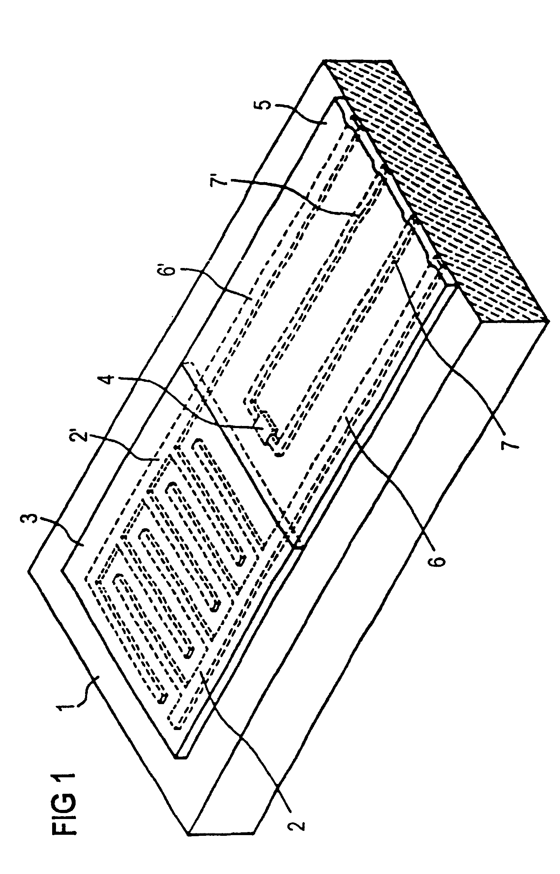

Das Methanmolekül besitzt aufgrund der sp3-Hybridisierung seiner Elektronenorbitale eine Tetraederstruktur. Es ist daher chemisch stabiler als die Moleküle organischer Lösungsmittel. Diese Eigenschaft nutzt die Erfindung, um das vergleichsweise reaktionsträge Methan in einem Lösungsmitteldämpfe enthaltenden Gasgemisch mit Hilfe des in Figur 1 schematisch dargestellten Sensors ohne störende Querempfindlichkeiten nachzuweisen.

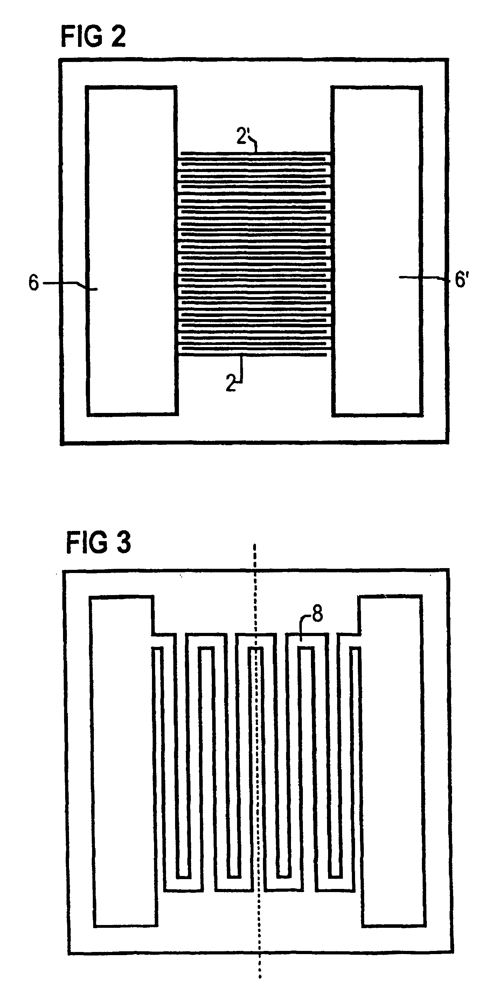

Der etwa 0,5 µm dicke Grundkörper 1 des Sensors besteht aus einem elektrisch isolierenden, temperaturstabilen Material wie Berylliumoxid, Aluminiumoxid oder Silizium. Auf ihm sind zwei eine Interdigitalstruktur bildende Platinelektroden 2, 2', eine die Kammelektroden 2, 2' leitend verbindende $Ga_2O_3$-Dünnschicht 3 als $CH_4$-empfindliches Element sowie ein Temperaturfühler 4 angeordnet. Eine $SiO_2$ Passivierung 5 soll die den beiden Kammelektroden 2, 2' und dem Temperaturfühler 4 zugeordneten Anschlußleitungen 6, 6' bzw. 7, 7' vor dem im Meßgas vorhandenen Sauerstoff abschirmen. Die Abmessungen der Kammelektroden 2, 2' hängen vom spezifischen Widerstand der mit Hilfe eines Sputter- oder Aufdampfverfahrens hergestellten und etwa 1 bis 2 µm dicken Sensorschicht 4 im gewünschten Temperaturbereich ab. So können die Elektroden 2, 2' der in Figur 2 maßstabsgetreu in Draufsicht dargestellten Interdigitalstruktur Dicken von 0,1 bis 10 µm, Breiten von 1 bis 1000 µm und Abstände von 1 bis 100 µm aufweisen.

Um die im Bereich von 700 °C $\leq$ T $\leq$ 1000 °C

liegende Betriebstemperatur einstellen und unabhängig von äußeren Einflüssen konstant halten zu können, wird der Sensor mit Hilfe einer auf der Rückseite des Grundkörpers 1 angeordneten, ggf. passivierten Widerstandsschicht aktiv beheizt. Diese in Figur 3 maßstabsgetreu in Draufsicht gezeigte Widerstandsschicht 8 besteht vorzugsweise aus Platin, Gold oder einer elektrisch leitenden Keramik.

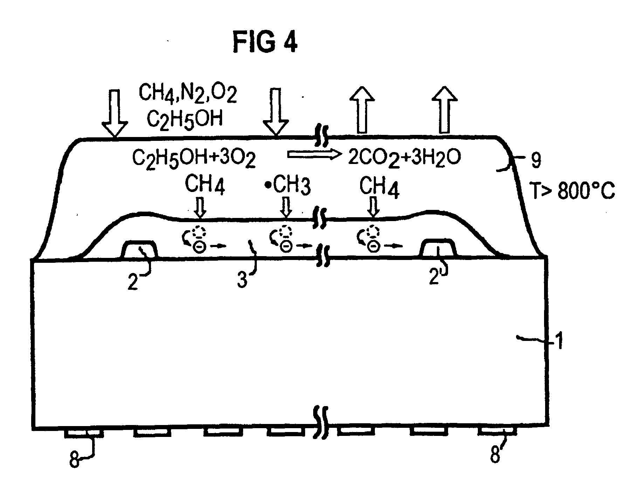

Die nur in Figur 4 dargestellte, das $CH_4$-sensitive Element 3 vollständig abdeckende, offenporöse Schicht 9 dient der Aufheizung des eindiffundierenden Meßgases. In ihr werden die vergleichsweise reaktiven organischen Lösungsmittel (Alkohol, Aceton) noch vor Erreichen der Dünnschicht 3 bei einer durch das Heizelement 8 vorgegebenen Temperatur $T \approx 800\ °C$ zu den die Leitfähigkeit des $Ga_2O_3$ nicht beeinflussenden Produkten CO, $CO_2$ oder $H_2O$ oxidiert. Das reaktionsträgere Methan wird demgegenüber kaum chemisch umgesetzt. Es gelangt an die Oberfläche der $Ga_2O_3$-Dünnschicht 3 und reagiert dort mit dem Sauerstoff des Metalloxids. Die dadurch entstehenden Sauerstoffleerstellen geben Elektronen an das Kristallgitter ab, so daß sich die Leitfähigkeit der $Ga_2O_3$-Dünnschicht 3 entsprechend der Methankonzentration im Meßgas erhöht. Aufgrund der in der etwa 1 bis 50 µm, insbesondere 10 µm dicken Deckschicht 9 herrschenden hohen Temperatur $T \geq 800\ °C$ wird das Zielgas durch Crackprozesse zum Teil auch in reaktivere Komponenten umgewandelt, welche die Leitfähigkeit der $Ga_2O_3$-Dünnschicht 3 stärker beeinflussen als Methan selbst.

Die.Deckschicht 9 besteht aus einem elektrisch nichtleitenden, temperaturbeständigen Material, wobei insbesondere $SiO_2$, SiN oder SiON (Siliziumoxinitrid) in polykristalliner oder amorpher Form sowie nichtleitende Keramiken (z. B. Keramiken auf der Basis der Metalloxide $Al_2O_3$ und BeO) oder Kombinationen der genannten Materialien in Betracht kommen. Das Aufbringen der Deckschicht 9 kann beispielsweise durch Anwendung von Siebdrucktechniken, Sedimentation, Ausfällung oder Vakuumbeschichtung erfolgen. Verbessert wird die Wirkung der Deckschicht 9, indem man sie mit einer Dispersion von Oxidationskatalysatoren wie Pt, Pd oder $Fe_2O_3$ versieht.

## 5. Meßergebnisse

Um festzustellen, ob und inwieweit die Vorerhitzung des Meßgases die gewünschte Oxidation der organischen Lösungsmittel (Alkohol bzw. Aceton) herbeiführt, wurde der in Figur 1 dargestellte $Ga_2O_3$-Sensor in der Probenkammer eines Hochtemperaturofens angeordnet und Lösungsmitteldämpfen ausgesetzt. Die dem Sensor zugeführte Heißluft enthielt in einer ersten Versuchsreihe 1000 ppm Äthanol, in einer zweiten Versuchsreihe 1000 ppm Aceton, wobei die Gastemperatur immer der Sensortemperatur entsprach. Meßgröße war die durch den Quotienten

$$R_{Luft}/R_{Äthanol}\ bzw.\ R_{Luft}/R_{Aceton}$$

| | |
|---|---|
| $R_{Luft}$: | Sensorwiderstand an Luft |
| $R_{Äthanol}$: | Sensorwiderstand an Luft + 1000 ppm Äthanol |
| $R_{Aceton}$: | Sensorwiderstand an Luft + 1000 ppm Aceton |

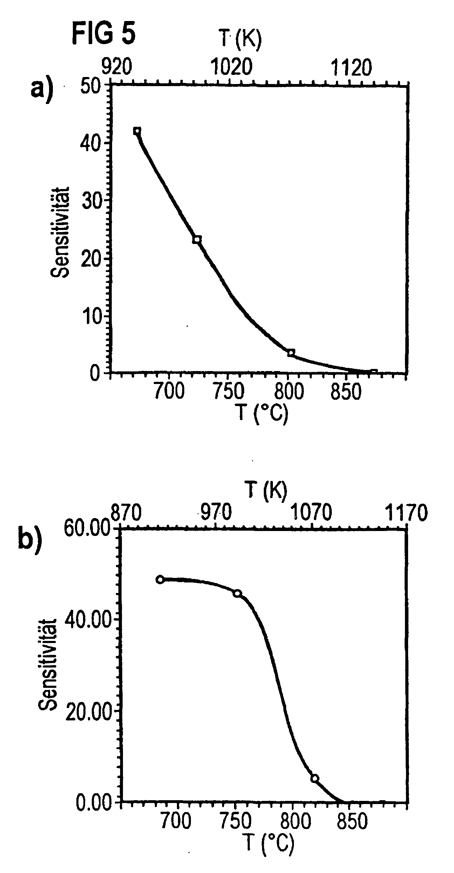

definierte Sensitivität in Abhängigkeit von der Gastemperatur (= Sensortemperatur). Die entsprechenden Meßkurven sind in Figur 5a und b dargestellt. Man erkennt deutlich, daß die Querempfindlichkeit der $Ga_2O_3$-Dünnschicht auf Äthanol und Aceton bei Temperaturen $T > 800\ °C$ nahezu vollständig verschwindet. Eine massenspektroskopische Analyse des durch die Probenkammer strömenden Gases bestätigte die vermutete Umsetzung des Alkohols in $CO_2$ und $H_2O$.

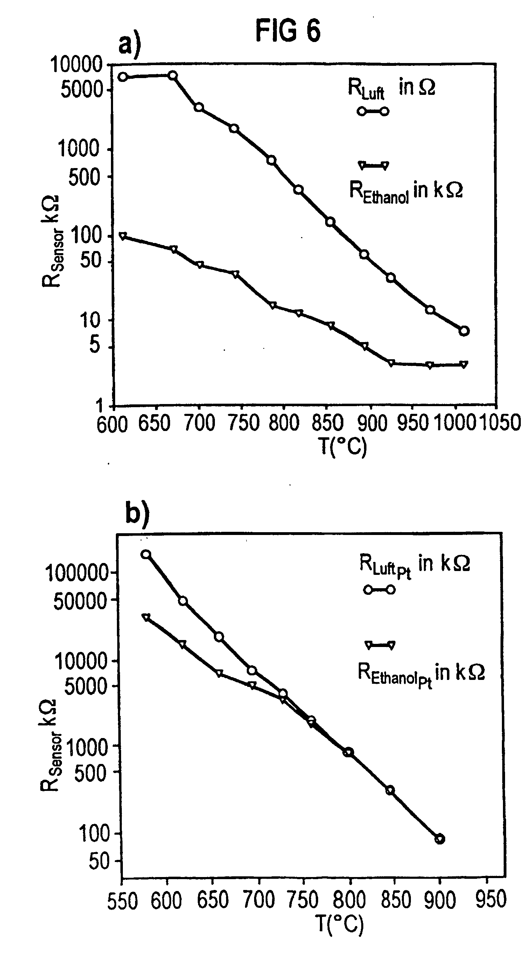

Die Oxidation der Lösungsmittel läßt sich auch durch eine katalytische Aktivierung der Sensoroberfläche erreichen. So zeigt die unbehandelte $Ga_2O_3$-Dünnschicht die erwartete Alkoholempfindlichkeit (s. Figur 6a). Die mit einer Pt-Dispersion behandelte $Ga_2O_3$-Dünnschicht spricht hingegen ab einer Temperatur $T > 750\ °C$ nicht mehr auf Äthanol an (Sättigungs- konzentration über einer Lösung 3 % Äthanol in Wasser).

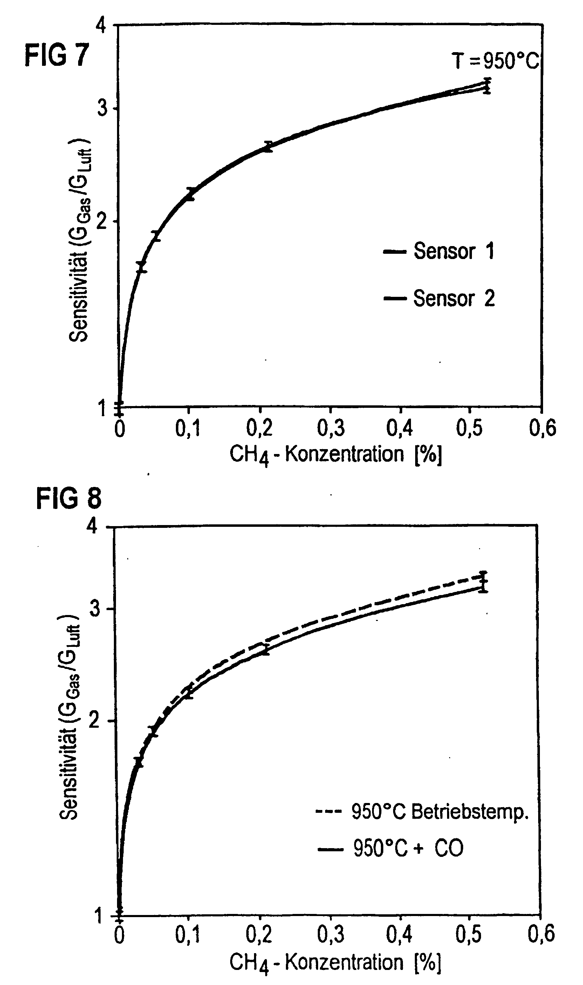

Wie die Figur 7 zeigt, reagiert der $Ga_2O_3$-Dünnschichtsensor sehr empfindlich auf Methan. So ändert sich der Leitwert um einen Faktor 3, selbst wenn die Luft nur 0,4 % Methan enthält. Auch eine deutlich über den MAK-Wert liegende Konzentration von 1000 ppm CO beeinflußt das Meßsignal nur unwesentlich.

## 6. Ausgestaltungen und Weiterbildungen der Erfindung

Die Erfindung beschränkt sich selbstverständlich nicht auf die oben beschriebenen Ausführungsbeispiele. So kann die der Erwärmung des Meßgases dienende Deckschicht 9 entfallen, wenn man den Sensor in einem den Gasaustausch ermöglichenden Gehäuse anordnet und dieses beheizt. Weiterhin ist es möglich, ein mit einem Heizmäander versehenes Plättchen parallel zur Sensoroberfläche anzuordnen. Der zwischen den beiden Elementen vorhandene Luftspalt gewährleistet die Erhitzung des Meßgases und den erforderlichen Gasaustausch. Von Vorteil kann es sein, die sensorseitige Oberfläche des Plättchens noch mit einer katalytisch aktiven Schicht bzw. Dispersion aus einem Edelmetall wie Pt oder Pd oder einem Nebengruppenmetalloxid wie $Fe_2O_3$ zu versehen.

Anstelle von $Ga_2O_3$ lassen sich insbesondere auch die sauerstoffsensitiven, halbleitenden Metalloxide $TiO_2$, $Fe_2O_3$, $CeO_3$, $SrTiO_3$, $Nb_2O_3$ oder $HfO_2$ verwenden, um deren von der Methankonzentration abhängigen Widerstand, Leitwert oder relative Permeabilität zu messen.

## 7. Literatur

/1/ DE 43 10 914 A1
/2/ WO 94/23 288

### Patentansprüche

1. Verfahren zum Nachweis von Methan in einem Gasgemisch mit den folgenden Schritten:

   - Erhitzen des Gasgemischs auf eine Temperatur T ≥ 800 °C ;
   - Heranführen des erhitzten Gasgemischs an ein sauerstoffempfindliches halbleitendes Metalloxid, dessen Temperatur T ≥ 800 °C beträgt und
   - Messung des Widerstands, der Leitfähigkeit oder der relativen Permeabilität des Metalloxids.

2. Verfahren nach Anspruch 1, **dadurch gekennzeichnet,** **daß** das Gasgemisch in einer das Metalloxid (3) abdeckenden, porösen Schicht (9) aufgeheizt wird.

3. Verfahren nach Anspruch 2, **dadurch gekennzeichnet,** **daß** eine aus einer nichtleitenden Keramik, aus polykristallinem oder amorphen $SiO_2$, SiN oder SiON oder aus Silikatgläsern bestehende Schicht (9) zur Aufheizung des Meßgases verwendet wird.

4. Verfahren nach einem der Ansprüche 1 bis 3, **dadurch gekennzeichnet,** **daß** das Metalloxid auf einer konstanten Temperatur gehalten wird, wobei die Temperatur im Bereich von 800 °C ≤ T < 1000 °C liegt.

5. Verfahren nach einem der Ansprüche 1 bis 4, **dadurch gekennzeichnet,** **daß** $Ga_2O_3$, $TiO_2$, $Fe_2O_3$, $CeO_3$, $SrTiO_3$, $Nb_2O_3$ oder $HfO_2$ als Metalloxid verwendet wird.

6. Verfahren nach einem der Ansprüche 1 bis 5, **dadurch gekennzeichnet,** **daß** die Schicht (9) oder das Metalloxid mit einer Dispersion eines Oxidationskatalysators versehen wird.

### Claims

1. Method for the detection of methane in a gas mixture, comprising the following steps:

   - heating the gas mixture to a temperature T ≥ 800°C;
   - guiding the heated gas mixture onto an oxygen-sensitive semiconducting metal oxide, the temperature of which is T ≥ 800°C, and
   - measuring the resistance, the conductivity or the relative permeability of the metal oxide.

2. Method according to Claim 1, **characterized in that** the gas mixture is heated in a porous layer (9) which covers the metal oxide (3).

3. Method according to Claim 2, **characterized in that** a layer consisting of a nonconductive ceramic, of polycrystalline or amorphous $SiO_2$, SiN or SiON or of silicate glasses is used to heat the measurement gas.

4. Method according to one of Claims 1 to 3, **characterized in that** the metal oxide is held at a constant temperature, the temperature lying in the range from 800°C ≤ T < 1000°C.

5. Method according to one of Claims 1 to 4, **characterized in that** the metal oxide used is $Ga_2O_3$, $TiO_2$, $Fe_2O_3$, $CeO_3$, $SrTiO_3$, $Nb_2O_3$ or $HfO_2$.

6. Method according to one of Claims 1 to 5, **characterized in that** the layer (9) or the metal oxide is provided with a dispersion of an oxidation catalyst.

### Revendications

1. Procédé de détention de méthane dans un mélange gazeux comprenant les stades suivants :

   - on chauffe le mélange gazeux à une température T ≥ 800° C ;
   - on fait passer le mélange gazeux chauffé sur un oxyde métallique semi-conducteur sensible à l'oxygène dont la température T est ≥ 800° C, et
   - on mesure la résistance, la conductivité ou la perméabilité relative de l'oxyde métallique.

2. Procédé suivant la revendication 1, **caractérisé** **en ce que** l'on chauffe le mélange gazeux dans une couche (9) poreuse recouvrant l'oxyde (3) métallique.

3. Procédé suivant la revendication 2, **caractérisé** **en ce que** l'on utilise, pour le chauffage du gaz à mesurer, une couche (9) en une céramique non conductrice en $SiO_2$ polycristallin ou amorphe en SiN ou SiON ou en verre au silicate.

4. Procédé suivant l'une des revendications 1 à 3, **caractérisé** **en ce que** l'on maintient l'oxyde métallique à une température constante, la température étant dans l'intervalle de 800° C $\leq$ T < 1000° C.

5. Procédé suivant l'une des revendications 1 à 4, **caractérisé** **en ce que** l'on utilise comme oxyde métallique $Ga_2O_3$, $TiO_2$, $Fe_2O_3$, $CeO_3$, $SrTiO_3$, $Nb_2O_3$, ou $HfO_2$.

6. Procédé suivant l'une des revendications 1 à 5, **caractérisé** **en ce que** l'on munit la couche (9) ou l'oxyde métallique d'une dispersion d'un catalyseur d'oxydation.

FIG 1

EP 0 743 515 B1

## FIG 2

## FIG 3

## FIG 4

$CH_4, N_2, O_2$
$C_2H_5OH$

$C_2H_5OH + 3O_2 \Rightarrow 2CO_2 + 3H_2O$

9

$CH_4$   •$CH_3$   $CH_4$

$T > 800°C$

2   3   2

1

8   8

**FIG 5**

a)

b)

FIG 6

FIG 7

$T = 950°C$

Sensor 1

Sensor 2

Sensitivität ($G_{Gas}/G_{Luft}$)

CH$_4$ - Konzentration [%]

FIG 8

Sensitivität ($G_{Gas}/G_{Luft}$)

--- 950°C Betriebstemp.

— 950°C + CO

CH$_4$ - Konzentration [%]